# EUROPEAN PATENT APPLICATION

(11) **EP 2 824 043 A1**
(43) Date of publication of application: **14.01.2015**
(21) Application number: 13175551.4
(22) Date of filing: 08.07.2013
(51) Int. Cl.: B65D 83/60, A61L 24/00

(54) **Hydrocolloid paste applicator**

(71) Applicant: OxMed International GmbH, 46446 Emmerich am Rhein (DE)
(72) Inventor: de Weert, Heleen, 7001 KJ Doetinchem (NL)
(74) Representative: Nederlandsch Octrooibureau

(57) **Abstract**

Combination of a hydrocolloid paste (5) and a container (1), the container (1) having a variable volume space (4) and an applicator (6) in communication with the variable volume space (4), the variable volume space (4) comprising the hydrocolloid paste (5) having a predetermined viscosity. The container (1) further comprises a pressure control device (8) providing a predetermined pressure on the variable volume space (4).

## Description

### Field of the invention

The present invention relates to a combination of a hydrocolloid paste and a container for applying the hydrocolloid paste. In a further aspect, the present invention relates to method of preparing the hydrocolloid paste.

### Prior art

International patent application WO 2012/168916 A2 discloses a pumping device for dispensing a fluid from a container comprising an actuator and a head part for dispensing the fluid from the container. The actuator comprises an outlet conduit and a first cylindrical member and the head part comprises a second cylindrical member which is arranged coaxially to the first cylindrical member. A folded membrane member with an inlet valve and an outlet valve is provided between the first and second cylindrical members, such that the first and second cylindrical members and the folded membrane member pro vide a variable pump chamber. The outlet valve is arranged relative to the actuator in such a manner that a suck-back chamber with a variable volume is provided between the outlet valve and the outlet conduit, which is simultaneously reduced as the pumping chamber is reduced.

The prior art container disclosed above does not seem to be appropriate for highly viscous and sticky substances such as a stoma paste, because the pumping force would become too great for manual operation. Furthermore, the pumping device provides an intermittent and pulsating application of the fluid in the container for each pumping stroke, making an homogenous and even distribution of the fluid more difficult.

### Summary of the invention

The present invention seeks to provide a stoma paste in a container which allows for ease of control whilst applying the paste.

According to the present invention, a combination of a hydrocolloid paste and a container according to the preamble defined above is provided, the container having a variable volume space and an applicator in communication with the variable volume space, the variable volume space comprising the hydrocolloid paste having a predetermined viscosity, the container further comprising a pressure control device providing a predetermined pressure on the variable volume space. The applicator of the present invention embodiments therefore eliminates excessive and repetitive squeezing by hand, as is customary when applying the hydrocolloid paste from a tube or the like as in present day applications.

In an embodiment, the pressure control device provides a substantially constant pressure on the variable volume space, thereby providing a substantially constant flow rate of stoma paste out of the container. The constant flow rate greatly facilitates a controlled, continuous and even application of the stoma paste to the skin and stoma, independent from the actual internal volume of the variable volume space.

In a further embodiment, the applicator comprises a nozzle which is arranged to allow the hydrocolloid paste to leave the container upon actuation of the nozzle. The applicator of the present invention therefore further eliminates excessive and repetitive squeezing by hand, avoiding repetitive strain injuries, tendon injuries and the like.

Advantageously, actuation of the nozzle may be accomplished by tilting or pushing the nozzle, which is a particularly convenient way of applying the stoma paste as this may be done with one hand or both hands in a gentle manner. The nozzle may be actuated by e.g. a thumb or index finger.

In an embodiment, the container is at least partly made of transparent material for providing visual information on the amount of stoma paste in the container.

In a further aspect, the present invention relates to a method of preparing a hydrocolloid paste, comprising adding a 20% dispersion of fumed silica in ethanol whilst mixing continuously; adding an adhesive matrix in the form of a film former in alcohol to the mixture whilst mixing continuously, and adding and dispersing hydrocolloid components and aloe vera powder whilst mixing continuously. This results in a hydrocolloid paste which can be used advantageously in combination with the container as described above.

In a further aspect, the present invention also relates to the use of a combination of a container and a hydrocolloid paste according to any one of the present invention embodiments for evening out skin prior to applying a plaster, e.g. a stoma plaster, wound plaster, etc.

### Short description of drawings

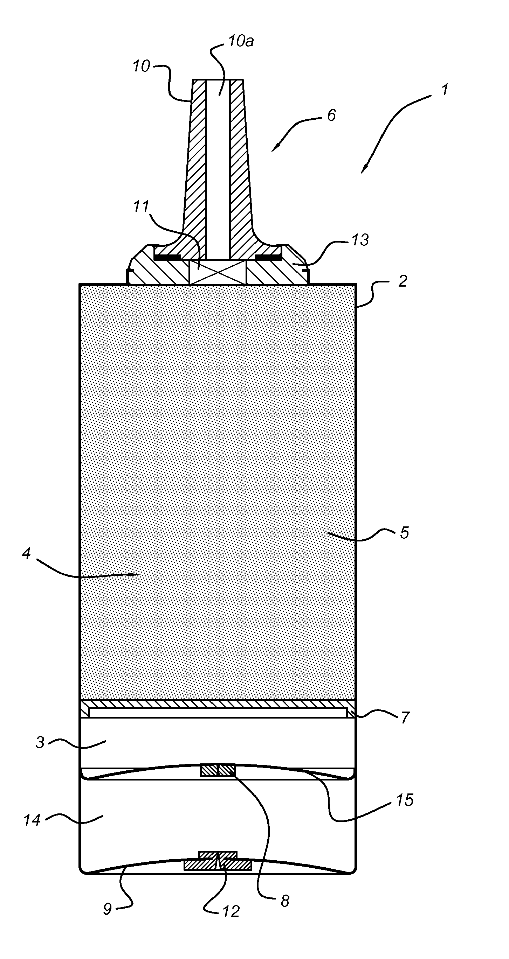

The present invention will be discussed in more detail hereinafter, using a number of exemplary embodiments with reference to the drawings, in which
Fig. 1 shows an embodiment of a combination of a hydrocolloid paste and a container according to the present invention.

### Detailed description of exemplary embodiments

Stoma paste is a caulking agent to fill gaps and creases between the skin and the skin barrier component of e.g. an ostomy appliance, or other medical applications, such as wound care. The main components of stoma paste are an adhesive matrix and a mixture of water-absorbing hydrocolloids as filler particles, in other words, a hydrocolloid paste. Important functions of the matrix are immediate adhesion on application (dry tack), flexibility and ease of removal. By absorbing moisture from the skin, the hydrocolloids aid in maintaining this adhesion throughout wear time (wet tack) as well as maintaining the skin in good condition.

Currently, stoma paste is available in tubes only. It is often difficult to squeeze the (full amount of) paste from these tubes due to the high viscosity and stickiness of the paste.

Fig. 1 shows an embodiment of a combination of a stoma paste 5 in a container 1, wherein the container 1 allows for ease of control during application of said paste 5. The container 1 has a variable volume space 4 comprising a stoma paste 5 having a predetermined viscosity, in particular a hydrocolloid paste 5. In an advantageous embodiment, the container 1 may be made of transparent material, such as a transparent plastic, e.g. Polyethylene terephthalate (PET), for visual inspection of the contents of the container 1.

The container 1 further comprises a plunger element 7 moveably disposed inside a bottle 2 determining the variable volume space 4 as a function of its position inside the bottle 2. A secondary variable volume space 3 is interposed between the plunger element 7 and an internal wall 15 fixedly connected to the bottle 2. Clearly, the sum of the variable volume space 4 and the secondary variable volume space 3 remains constant independent of the position of the plunger element 7 inside the bottle 2.

The container 1 further comprises a pressure control device 8 (PCD) disposed on the internal wall 15 and adapted to regulate a gas pressure inside the secondary variable volume space 3 for actuating the plunger element 7.

The container 1 also comprises a pressure chamber 14 interposed between the internal wall 15 and a bottom 9 of the bottle 2, wherein the pressure chamber 14 is in communication with the secondary variable volume space 3 through the pressure control device 8. The pressure chamber 14 can be pressurized, e.g. at 5 bar or higher, by means of a bottom plug 12 in the bottom 9.

In advantageous embodiments, the pressure control device 8 regulates the gas pressure inside the secondary variable volume space 3 toward a substantially constant gas pressure, wherein the gas pressure in the pressure chamber 14 is larger than the pressure in the secondary variable volume space 3. In an embodiment, the pressure in the secondary variable volume space 3 is approximately 2 to 3 bar. A substantially constant gas pressure in the secondary variable volume space 3 provides a constant flow rate of stoma paste 5 out of the container 1. In typical embodiments, the gas used in the pressure chamber 14 and secondary variable volume space 3 is air.

The container 1 further comprises an applicator 6 for applying the stoma paste 5 in a controlled manner, wherein the applicator 6 is provided with a mounting cup 13 fixedly mounted on the bottle 2 and a nozzle 10 disposed on said mounting cup 13. The mounting cup 13 may be made of a metal, e.g. aluminium, and the nozzle 10 may be made of a plastic, e.g. Polyethylene (PE). The nozzle 10 comprises a nozzle channel 10a through which the stoma paste 5 can be dispensed.

In an advantageous embodiment, the mounting cup 13 comprises a valve 11 that is opened by pushing or tilting the nozzle 10 of the applicator 6, thereby creating a passageway from the variable volume space 4, through the valve 11 and nozzle 10. When the valve 11 is opened, the plunger element 7 is able to push the stoma paste 5 out of the container 1 as a result of a sufficiently large (over)pressure in the secondary variable volume space 3. Pushing or tilting the nozzle may be accomplished by e.g. a thumb or index finger.

It is readily apparent that the viscosity and stickiness of the stoma paste 5 in the variable volume space 4 determines a required pressure in the secondary variable volume space 3 for dispensing the stoma paste 5.

According to the invention, there is no need to squeeze the container 1 to apply the stoma paste 5. Instead, the plunger element 7 is moved by a sufficiently large, substantially constant gas pressure in the secondary variable volume space 3. The container 1 is advantageous for highly viscous, sticky paste-like substances as is it able to completely empty the contents of the variable volume space 4. In particular, the geometry of the bottle 2, the applicator 6 and plunger element 7 are adapted to allow for a complete emptying the container 1. Medical personnel will find the combination of a stoma paste 5 and the container 1 particularly advantageous as it prevents repetitive strain injuries due to excessive and frequent squeezing.

Often, in present day applications, a bit of paste is applied from a tube, and the tube is put aside for a while, after which a further bit of paste is applied. Usually, drying out of the exposed bit of paste resulted in even further difficulty for squeezing out the paste, or even a total blockage resulting in throwing away the entire tube with remaining paste. Surprisingly it was found that the present invention embodiments of the combination of hydrocolloid paste and container showed a much lower tendency to dry out during actual use, providing additional cost savings by preventing disposal of tubes blocked by dried out paste. Surprisingly, dispensing can be continued several days after use. The container 1 can be emptied completely.

In view of the above, the present invention can thus be summarized as a combination of a hydrocolloid paste 5 and a container 1, the container 1 having a variable volume space 4 and an applicator 6 in communication with the variable volume space 4, the variable volume space 4 comprising the hydrocolloid paste 5 having a predetermined viscosity, the container 1 further comprising a pressure control device 8 providing a predetermined pressure on the variable volume space 4.

In an embodiment, the pressure control device 8 provides a substantially constant pressure on the variable volume space 4, thereby providing a substantially constant flow rate of stoma paste 5 out of he container 1. The constant flow rate greatly facilitates a controlled, continuous and even application of the stoma paste 5.

In a further embodiment, the applicator 6 comprises a nozzle 10 which is arranged to allow the hydrocolloid paste 5 to leave the container 1 upon actuation of the nozzle 10. The applicator of the present invention therefore eliminates excessive and repetitive squeezing by hand, avoiding repetitive strain injuries, tendon, muscle injuries and the like.

Advantageously, actuation of the nozzle 10 may be accomplished by tilting or pushing the nozzle 10, which is a particularly convenient way of applying the stoma paste 5 as this may be accomplished with one hand or both hands in a gentle manner. The nozzle 10 may be actuated by e.g. a thumb or index finger.

In an embodiment, the container is at least partly made of transparent material for providing visual information on the amount of stoma paste in the container.

The composition of the stoma paste as described hereinafter and used in conjunction with the container 1 as described above, has similarities with known hydrocolloid pastes, such as protective adhesive pastes, wherein the ConvaTec Stomahesive Paste is a well-known type of commercially available hydrocolloid paste. The ConvaTec matrix is a film forming resin in alcohol, which is commercially available under the trade name Gantrez ES-425.

Hydrocolloids come in different types, shapes and sizes; each of them having unique properties. Varying the types and amounts allows for control of parameters such as absorption rate and viscosity.

The absorption rate of ConvaTec is very favourable according to many nurses and end-users. A further commercially available hydrocolloid paste, Eakin Cohesive paste, has a very loose matrix with much faster and higher moisture absorption. The hydrocolloid paste according to the present invention embodiments (ForTe paste) was subjected to a comparative test with both ConvaTec and Eakin. Samples were prepared (ring shaped, approximately 5g and approximately 10 cm₂ surface area) and placed on sponges which were soaked in tap water (kept at body temperature). The water uptake of the samples was registered.

Initially, the ForTe paste absorbs slower than ConvaTec but the next day there was an approach. There were no striking visual differences between these pastes; both remained intact during the entire test. In contrast, Eakin absorbs much faster, resulting in disintegration within several hours. Once incorporated into the paste, microscopic investigation of the hydrocolloid particles is still possible. This is facilitated by diluting the paste in ethanol. In this way, a comparison was made between ForTe and ConvaTec paste. It appeared that the gelatine particles in the ConvaTec formulation were smaller. Another gelatine grade was sourced with a more similar particle size.

In specific embodiments, the hydrocolloid paste comprises an additional film former (PVP/VA copolymer) for high elasticity and fumed silica (silicon dioxide) as a thickener. The use of fumed silica is additionally beneficial for the resistance to body fluids.

For aloe vera a highly concentrated, pure, spray dried powder (prepared from aloe vera gel) was selected. Although in a different matrix, the European patent EP0928207 discloses that aloe vera powder has been found to prevent and alleviate skin problems for users of ostomy appliances.

In a first series of tests, all powders for the present invention hydrocolloid paste were dry blended. This blend was added to the Gantrez solution while mixing. Mixing is continued after the addition of ethanol until the paste is homogenous. The resulting paste looked, felt and behaved quite similar to ConvaTec during application. This was confirmed in a blind comparative test by several stoma nurses.

However, after a while it appeared that phase separation occurred: the hydrocolloid particles settled, leaving a clearer layer of liquid on top of the paste.

Further investigation revealed several points of attention regarding the stability of the paste:
a) The thickening mode of action of the fumed silica (e.g. AEROSIL 200 Pharma colloidal silicon dioxide) is through formation of a three-dimensional network of AEROSIL aggregates in a liquid. Embedding the hydrocolloid particles in this network would reduce their tendency to settle. It is assumed that the formation of a network is disturbed when AEROSIL is added to the powder blend first. Therefore, this network is achieved before the remaining powders are added in one of the present invention embodiments.
b) The settling velocity of the hydrocolloid particles may be reduced by using finer powders. The gelatine particles in the first series of tests appeared to be much larger than the ones present in ConvaTec. A gelatine grade with a smaller particle size was then selected as alternative component for the hydrocolloid paste.
c) The PVP/VA copolymer appeared to have no added value in the formulation. It was decided to omit this ingredient in a further embodiment, leaving room for the other components.

In the second series of tests, a dispersion of 20% AEROSIL in ethanol is prepared first. This yields a highly viscous thixotropic (shear thinning) gel: it has a rather high viscosity if left to rest but the viscosity goes down as soon as shear is applied. Mixing is continued while first Gantrez is added and then the remaining powder blend. The paste from the second series looks, feels and behaves quite similar to ConvaTec during application, and shows no sign of phase separation.

So, according to a further aspect, the present invention provides an efficient method of preparing a hydrocolloid paste. In an embodiment, the method comprises adding a 20% dispersion of fumed silica in ethanol whilst mixing continuously; adding an adhesive matrix in the form of a film former in alcohol to the mixture whilst mixing continuously; adding and dispersing hydrocolloid components and aloe vera powder whilst mixing continuously.

In an embodiment, the prepared hydrocolloid paste comprises between 4 and 16 weight% ethanol, e.g. 8 weight%. In a further embodiment, the prepared hydrocolloid paste comprises between 40-70 weight% of adhesive matrix, e.g. 52 weight%. In an even further embodiment, the prepared hydrocolloid paste comprises between 0.5 and 4 weight % of fumed silica, e.g. 2 weight%.

Advantageously, the hydrocolloid components may comprise pectin, gelatin and sodium carboxymethylcellulose in equal quantities, e.g. each substantially 12.5 weight%. Also, an additional skin care component may be added, wherein the prepared hydrocolloid paste comprises between 0.25 and 1 weight% of aloe vera powder, e.g. 0.5%.

In typical embodiments, the hydrocolloid paste consists of a film former in alcohol, one or more hydrocolloid substances, fumed silica, an aloe vera based component, and alcohol.

As described above, the present invention also relates to the use of a combination of a container and a hydrocolloid paste according to any one of the present invention embodiments for evening out skin prior to applying a plaster, e.g. a stoma plaster, wound plaster, etc.

The present invention embodiments have been described above with reference to a number of exemplary embodiments as shown in the drawings. Modifications and alternative implementations of some parts or elements are possible, and are included in the scope of protection as defined in the appended claims.

## Claims

1. Combination of a hydrocolloid paste and a container, the container (1) having a variable volume space (4) and an applicator (6) in communication with the variable volume space (4), the variable volume space (4) comprising the hydrocolloid paste having a predetermined viscosity,
the container (1) further comprising a pressure control device (8) providing a predetermined pressure on the variable volume space (4).

2. Combination according to claim 1, wherein the pressure control device (8) provides a substantially constant pressure on the variable volume space (4).

3. Combination according to claim 1 or 2, wherein the applicator (6) comprises a nozzle (10) which is arranged to allow the hydrocolloid paste to leave the container (1) upon actuation of the nozzle (10).

4. Combination according to claim 3, wherein actuation of the nozzle (10) is accomplished by tilting or pushing the nozzle (10).

5. Combination according to any one of claims 1-4, wherein the container (1) is at least partly made of transparent material.

6. Combination according to any one of claims 1-5, wherein the hydrocolloid paste is a stoma paste comprising an adhesive matrix and a mixture of water-absorbing hydrocolloids as filler particles.

7. Combination according to claim 6, wherein the adhesive matrix is a film former in alcohol, and the hydrocolloid paste further comprises fumed silica and alcohol.

8. Combination according to claim 6 or 7, wherein the hydrocolloid paste further comprises an aloe vera based component.

9. Method of preparing a hydrocolloid paste, comprising
adding a 20% dispersion of fumed silica in ethanol whilst mixing continuously; adding an adhesive matrix in the form of a film former in alcohol to the mixture whilst mixing continuously;
adding and dispersing hydrocolloid components and aloe vera powder whilst mixing continuously.

10. Method according to claim 9, wherein the prepared hydrocolloid paste comprises between 4 and 16 weight% ethanol, e.g. 8 weight%.

11. Method according to claim 9 or 10, wherein the prepared hydrocolloid paste comprises between 40-70 weight% of adhesive matrix, e.g. 52 weight%.

12. Method according to claim 9, 10 or 11 wherein the prepared hydrocolloid paste comprises between 0.5 and 4 weight % of fumed silica, e.g. 2 weight%.

13. Method according to any one of claims 8-12, wherein the prepared hydrocolloid paste comprises between 0.25 and 1 weight% of aloe vera powder, e.g. 0.5%.

14. Hydrocolloid paste consisting of a film former in alcohol, one or more hydrocolloid substances, fumed silica, an aloe vera based component, and alcohol.

15. Use of a combination of a container and a hydrocolloid paste according to any one of claims 1-8 for evening out skin prior to applying a plaster.
